# NOUVEAU FASCICULE DE BREVET EUROPEEN

(11) **EP 0 966 252 B2**
(45) Date de publication et mention de la décision concernant l'opposition: **07.10.2009**
(45) Mention de la délivrance du brevet: 03.11.2004
(21) Numéro de dépôt: 98939696.5
(22) Date de dépôt: 16.07.1998
(51) Int. Cl.: A61Q 5/10, A61K 8/41, A61K 8/49

(54) **COMPOSITION POUR LA TEINTURE D'OXYDATION DES FIBRES KERATINIQUES COMPRENANT DU 2-CHLORO 6-METHYL 3-AMINOPHENOL ET UNE BASE D'OXYDATION, ET PROCEDE DE TEINTURE**
2-CHLOR-6-METHYL-3-AMINOPHENOL UND EINE OXIDATIONSBASE ENTHALTENDE ZUSAMMENSETZUNG ZUR OXIDATIVEN FÄRBUNG VON KERATINFASERN UND FÄRBEVERFAHREN
OXIDATION DYEING COMPOSITION FOR KERATIN FIBRES COMPRISING 2-CHLORO 6 METHYL 3-AMINOPHENOL AND AN OXIDATION BASE, AND DYEING METHOD

(30) Priorité: 01.09.1997 FR 9710854
(43) Date de publication de la demande: 29.12.1999
(60) Demande divisionnaire: 03291367.5
(73) Titulaire: L'ORÉAL, 75008 Paris (FR)
(72) Inventeur: AUDOUSSET, Marie-Pascale, F-92600 Asnières (FR)
(74) Mandataire: Goulard, Sophie
(86) Numéro de dépôt international: PCT/FR1998/001562
(87) Numéro de publication internationale: WO 1999/011230

(56) Documents cités:
- EP-A- 0 039 030
- EP-A- 0 591 329
- EP-A- 0 728 465
- EP-A1- 0 360 644
- EP-A1- 0 634 164
- WO-A-92/04883
- WO-A-96/15765
- WO-A-97/11674
- WO-A1-92/13824
- WO-A1-92/22525
- WO-A1-97/31886
- WO-A1-98/38175
- DE-A- 4 122 748
- DE-A- 4 205 329
- DE-B- 1 939 063
- FR-A- 2 687 399

## Description

La présente invention a pour objet une composition pour la teinture d'oxydation des fibres kératiniques, en particulier des fibres kératiniques humaines telles que les cheveux, comprenant du 2-chloro 6-méthyl 3-aminophénol à titre de coupleur, en association avec au moins une base d'oxydation convenablement sélectionnée, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols ou encore des composés hétérocycliques tels que des dérivés de pyrimidine, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec les bases d'oxydation en les associant à des coupleurs ou modificateurs de coloration convenablement choisis, ces derniers pouvant être notamment parmi des métadiamines aromatiques, des métaaminophénols, des métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet allemand DE 3 016 008 des compositions pour la teinture d'oxydation des fibres kératiniques contenant à titre de coupleur du 2-chloro 6-méthyl 3-aminophénol ou du 2-méthyl 5-chloro 3-aminophénol, en association avec des bases d'oxydation classiquement utilisées pour la teinture d'oxydation, telles que par exemple certaines paraphénylènediamines, du para-aminophénol ou encore de la tétraaminopyrimidine. De telles compositions ne sont cependant pas entièrement satisfaisantes notamment du point de vue de la tenue des colorations obtenues vis à vis des diverses agressions que peuvent subir les cheveux et en particulier vis à vis des shampooings et des déformations permanentes.

Il a également été proposé, dans les demandes de brevet WO 96/15765 et WO 96/15766, des compositions pour la teinture d'oxydation des fibres kératiniques contenant l'association spécifique du 2-chloro 6-méthyl 3-aminophénol à titre de coupleur et de bases d'oxydation particulières comme la 2-β-hydroxyéthyl paraphénylènediamine ou certains para-aminophénols particuliers comme par exemple le 3-méthyl 4-aminophénol, le 2-allyl 4-aminophénol ou bien encore le 2-aminométhyl 4-aminophénol. De telles compositions ne sont cependant pas non plus entièrement satisfaisantes notamment du point de vue de la puissance des colorations obtenues.

Il a été proposé, dans la demande EP 0 728 465, des compositions pour la teinture d'oxydation des fibres kératiniques comprenant une base d'oxydation choisie parmi les para-phénylènodiamines et les bis-phénylalkylènediamines, un premier coupleur choisi parmi des méta-aminophénols de formule déterminée et la 6-hydroxy indoline à titre de second coupleur.

Il a été proposé, dans la demande WO 9 711 674, des compositions pour la teinture d'oxydation des fibres kératiniques comprenant une base d'oxydation choisie parmi les para-phénylènediamines et les bis-phénylalkylènediamines, un premier coupleur choisi parmi des méta-aminophénols de formule déterminée et la 4-hydroxy indoline à titre de second coupleur.

Or, la demanderesse vient maintenant de découvrir qu'il est possible d'obtenir de nouvelles teintures puissantes et particulièrement résistantes aux diverses agressions que peuvent subir les cheveux, en associant du 2-chloro 6-méthyl 3-aminophénol et au moins une base d'oxydation convenablement sélectionnée.

Cette découverte est à la base de la présente invention.

L'invention a donc pour premier objet une composition telle que définie à la revendication 1.

Cette composition peut de plus contenir des bases d'oxydation additionnelles suivantes :
(a) les paraphénylènediamines de formule (I) suivante, et leurs sels d'addition avec un acide : 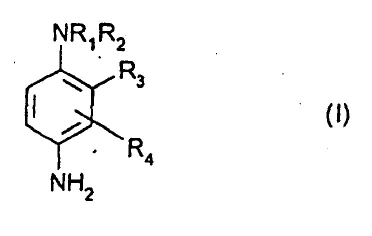 dans laquelle :
   - R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
   - R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
   - R₃ représente un atome d'hydrogène, un radical alkyle en C₁-C₄, monohydroxyalcoxy en C₁-C₄, un radical mésylaminoalcoxy en C₁-C₄, acétylaminoalcoxy en C₁-C₄, carbamoylaminoalcoxy en C₁-C₄ ou alkyle en C₁-C₄ substitué par un groupement azoté ;
   - R₄ représente un atome d'hydrogène ou un radical alkyle en C₁-C₄ ;
   étant entendu que :
   - au moins un des radicaux R₁, R₂, R₃ et R₄ est différent d'un atome d'hydrogène,
   - lorsque les radicaux R₁, R₂ et R₄ représentent simultanément un atome d'hydrogène alors le radical R₃ est différent d'un radical méthyle,
   - lorsque les radicaux R₁, R₂ et R₃ représentent simultanément un atome d'hydrogène et que le radical R₄ occupe la position 6, alors R₄ est différent d'un radical méthyle,
   - lorsque l'un des radicaux R₁ et R₂ représente un radical alkyle en C₁-C₄ ou monohydroxyalkyle en C₁-C₄, et que l'autre radical R₁ ou R₂ représente un atome d'hydrogène, alors au moins un des radicaux R₃ et R₄ est différent d'un atome d'hydrogène ;
   - lorsque R₁ et R₂ représentent simultanément un radical monohydroxyalkyle en C₁-C₄, alors au moins un des radicaux R₃ et R₄ est différent d'un atome d'hydrogène ;
c) les pyrazolo-[1,5-a]-pyrimidines de formule (III) suivante, leurs sels d'addition avec un acide ou avec une base et leurs formes tautomères, lorsqu'il existe un équilibre tautomérique : 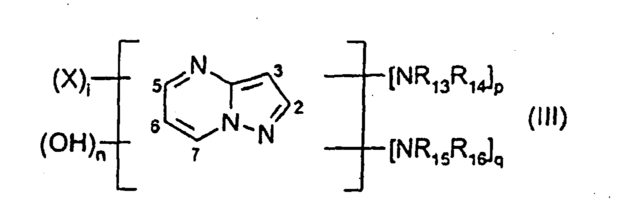 dans laquelle :
   - R₁₃, R₁₄, R₁₅ et R₁₆, identique ou différents désignent, un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical aminoalkyle en C₁-C₄ (l'amine pouvant être protégée par un radical acétyle, uréido ou sulfonyle), un radical (C₁-C₄)alkylamino alkyle en C₁-C₄, un radical di-[(C₁-C₄)alkyl] amino alkyle en C₁-C₄ (les radicaux dialkyles pouvant former un cycle carboné ou un hétérocycle à 5 ou 6 chaînons), un radical hydroxy(C₁-C₄)alkyl- ou di-[hydroxy(C₁-C₄) alkyl]-amino alkyle en C₁-C₄ ;
   - les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄(les dialkyles pouvant former un cycle carboné ou un hétérocycle à 5 ou 6 chaînons), un radical hydroxy(C₁-C4)alkyl ou di-[hydroxy(C₁-C₄)alkyl]amino alkyle en C₁-C₄, un radical amino, un radical (C₁-C₄)alkyl- ou di-[(C₁-C₄)alkyl]amino ; un atome d'halogène, un groupe acide carboxylique, un groupe acide sulfonique ;
   - i vaut 0, 1, 2 ou 3 ;
   - p vaut 0 ou 1 ;
   - q vaut 0 ou 1 ;
   - n vaut 0 ou 1 :
   sous réserve que :
   - la somme p + q est différente de 0 ;
   - lorsque p + q est égal à 2, alors n vaut 0 et les groupes NR₁₃R₁₄ et NR₁₅R₁₆ occupent les positions (2,3) ; (5,6) ; (6,7) ; (3,5) ou (3,7) ;
   - lorsque p + q est égal à 1 alors n vaut 1 et le groupe NR₁₃R₁₄ (ou NR₁₅R₁₆) et le groupe OH occupent les positions (2,3) ; (5,6) ; (6,7) ; (3,5) ou (3,7) ;
d) les orthoaminophénols ;
e) les diamino-pyridines ;
ladite composition étant exempte d'un coupleur additionnel qui serait choisi parmi la 4-hydroxy indoline, la 6-hydroxy indoline et leurs sels d'addition avec un acide.

La composition de teinture d'oxydation conforme à l'invention permet d'obtenir des colorations puissantes aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes). Ces propriétés sont particulièrement remarquables notamment en ce qui concerne la résistance des colorations vis à vis des shampooings et des déformations permanentes.

Parmi les radicaux alkyle en C₁-C₄ et alcoxy en C₁-C₄ des formules (I), (II) et (III) ci-dessus, on peut citer notamment les radicaux méthyle, éthyle, propyle, méthyloxy et éthyloxy.

Parmi les groupements azotés des formules (I) et (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus utilisables à titre de base d'oxydation dans les compositions tinctoriales conformes à l'invention, on peut plus particulièrement citer la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, le 4-amino 1-N,N-diéthylamino 3-méthyl benzène, la 4-amino 1-N,N-bis-(p-hydroxyéthyl)amino 3-méthyl benzène, la 2-isopropyl paraphénylènediamine, la 2-n-propyl paraphénylènediamine, la 2-β-hydroxyéthyloxy paraphénylènediamine, le N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(β-méthoxyéthyl)amino paraphénylènediamine, la 2-β-acétylaminoéthyloxy paraphénylènediamine et leurs sels d'addition avec un acide.

Parmi les paraphénylènediamines de formule (I) ci-dessus, on préfère plus particulièrement la 2,6-diméthyl paraphénylènediamine, la 2-n-propyl paraphénylènediamine, la 2-β-hydroxyéthyloxy paraphénylènediamine, la N-(β-méthoxyéthyl)amino paraphénylènediamine, la 2-β-acétylaminoéthyloxy paraphénylènediamine et leurs sels d'addition avec un acide.

Parmi les bases doubles de formule (II) ci-dessus utilisables à titre de base d'oxydation dans les compositions tinctoriales conformes à l'invention, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (II), le N,N'-bis-(β-hydroxyéthyl) N,N'-bis-(4'-aminophényl) 1,3-diamino propanol et ses sels d'addition avec un acide sont particulièrement préférés.

Lorsque les pyrazolo-[1,5-a]-pyrimidines de formule (III) ci-dessus sont telles qu'elles comportent un groupe hydroxyle sur l'une des positions 2, 5 ou 7 en α d'un atome d'azote, il existe un équilibre tautomérique représenté par exemple par le schéma suivant :

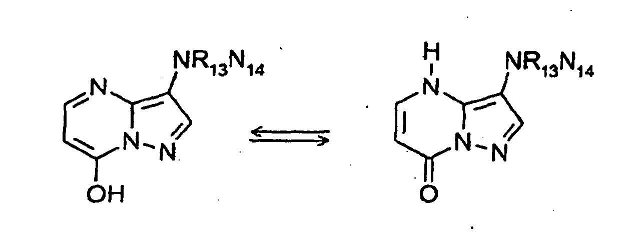

Parmi les pyrazo)o-[1,5-a]-pyrimidines de formule (III), utilisables à titre de base d'oxydation dans les compositions conformes à l'invention, on peut notamment citer :
- la pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)-amino]-éthanol
- le 2-[(7-Amino-pyrazolo[1,5-a]pyrimidin-3-yl)-(2-hydroxy-éthyl)amino]-éthanol
- la 5,6-diméthyl pyrazolo-[1,5-a]-pyrimidine-3,7-diamine ;
- ia 2,6-diméthyl pyrazolo-[1,5-a]-pyrimidine-3,7-diamine ;
- la 2, 5, N 7, N 7-tetraméthyl pyrazolo-[1,5-a]-pyrimidine-3,7-diamine ;
et leurs sels d'addition et leurs formes tautomères, lorsqu'il existe un équilibre tautomérique.

Les pyrazolo-[1,5-a]-pyrimidines de formule (III) conformes à l'invention peuvent être préparées par cyclisation à partir d'un aminopyrazole selon les synthèses décrites dans les références suivantes :
- EP 628559 BEIERSDORF-LILLY
- R. Vishdu, H. Navedul, Indian J. Chem., 34b (6), 514, 1995.
- N.S. Ibrahim, K.U. Sadek, F.A. Abdel-AI, Arch. Pharm., 320, 240, 1987.
- R.H. Springer, M.B. Scholten, D.E. O'Brien, T. Novinson, J.P. Miller, R.K. Robins, J. Med. Chem., 25, 235, 1982.
- T. Novinson, R.K. Robins, T.R. Matthews, J. Med. Chem., 20, 296, 1977.
- US 3907799 ICN PHARMACEUTICALS

Les pyrazolo-[1,5-a]-pyrimidines de formule (III) conformes à l'invention peuvent également être préparées par cyclisation à partir d'hydrazine selon les synthèses décrites dans les références suivantes :
- A. McKillop et R.J. Kobilecki, Heterocycles, 6(9), 1355, 1977.
- E. Alcade, J. De Mendoza, J.M. Marcia-Marquina, C. Aimera, J. Elguero, J. Heterocyclic Chem., 11(3), 423, 1974.
- K. Saito, I. Hori, M. Higarashi, H. Midorikawa, Bull. Chem. Soc. Japan, 47(2), 476,1974.

Parmi les orthoaminophénols utilisables à titre de base d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les diamino-pyridines utilisables à titre de base d'oxydation dans les compositions tinctoriales conformes à l'invention, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Le 2-chloro 6-méthyl 3-aminophénol et/ou le ou ses sels d'addition avec un acide, représentent de préférence de 0,0001 à 5 % en poids environ du poids total de la composition tinctoriale, et encore plus préférentiellement de 0,005 à 3 % en poids environ de ce poids.

La ou les bases d'oxydation présentes dans la composition conforme à l'invention et/ou leurs sels d'addition avec un acide représentent de préférence de 0,0005 à 12 % en poids environ du poids total de la composition tinctoriale, et encore plus préférentiellement de 0,005 à 6 % en poids environ de ce poids.

Les compositions tinctoriales conformes à l'invention peuvent contenir d'autres coupleurs différents du 2-chloro 6-méthyl 3-aminophénol et/ou des colorants directs notamment pour modifier les nuances ou les enrichir en reflets.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates. Les sels d'addition avec une base utilisables dans le cadre des compositions tinctoriales de l'invention (bases d'oxydation) sont notamment ceux obtenus avec la soude, la potasse, l'ammoniaque ou les amines.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. Le solvant organique est de préférence choisi parmi les alcanols inférieurs en C₁-C₄, tels que l'éthanol et l'isopropanol; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ et encore plus préférentiellement entre 5 et 11 environ. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 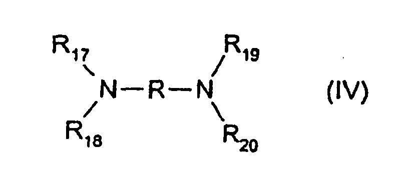 dans laquelle R est un reste propylène éventuellement substitué par un groupement hydroxyle ou un radical alkyle en C₁-C₄ ; R₁₇, R₁₈, R₁₉ et R₂₀, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à l'association conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### EXEMPLE 1 DE TEINTURE

On a préparé la composition tinctoriale, conforme à l'invention, suivante (teneurs en grammes) :

| **EXEMPLE** | **1** |
|---|---|
| 2-chloro 6-méthyl 3-aminophénol (coupleur) | 0,471 |
| Tétrachlorhydrate de N,N'-bis-(β-hydroxyéthyly)-N,N'-bis-(4'-aminophényl) 1,3-diamino propanol (base d'oxydation) | 1,518 |
| Support de teinture commun n°2 | (***) |
| Support de teinture commun n°3 | - |
| Eau déminéralisée q.s.p. | 100 g |

| | |
|---|---|
| (***) support de teinture commun n°2 : - Ethanol à 96 ° 18 g - Métabisulfite de sodium en solution aqueuse à 35 % 0,68 g - Sel pentasodique de l'acide diéthylènetriaminopentacétique 1,1 g - Ammoniaque à 20 % de NH₃ 10 g | |

Au moment de l'emploi, on a mélangé la composition tinctoriale ci-dessus avec une quantité égale en poids d'une composition oxydante constituée par une solution d'eau oxygénée à 20 volumes (6 % en poids).

La composition résultante a été appliquée pendant 30 minutes sur des mèches de cheveux gris naturels à 90 % de blancs. Les mèches de cheveux ont ensuite été rincées, lavées avec un shampooing standard puis séchées.

La nuance obtenue figure dans le tableau ci-dessous:

## Revendications

1. Composition pour la teinture d'oxydation des fibres kératiniques humaines telles que les cheveux, **caractérisée par le fait qu'**elle comprend, dans un milieu approprié pour la teinture :
- du 2-chloro 6-méthyl 3-aminophénol et/ou au moins l'un de ses sels d'addition avec un acide, à titre de coupleur,
- et au moins une base d'oxydation choisie parmi les bases doubles de formule (II) suivantes et leurs sels d'addition avec un acide : 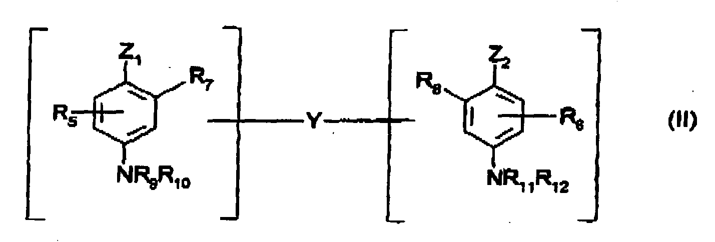 dans laquelle:
- Z₁ et Z₂, identiques ou différents, représentent un radical -NH₂ pouvant être substitué par un radical alkyle en C₁-C₄ ou par un bras de liaison Y;
- le bras de liaison Y représente une chaîne alkylène comportant de 1 à 14 atomes de carbone, linéaire ou ramifiée pouvant être interrompue par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄ ou aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule;
ladite composition étant exempte d'un coupleur additionnel qui serait choisi parmi la 4-hydroxy indoline, la 6-hydroxy indoline et leurs sels d'addition avec un acide.

2. Composition selon la revendication 1, **caractérisée par le fait que** les bases doubles de formule (II) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthytènediamine, la N,N'-bis-(4-aminophényl) tétraméthylènediamine, la N,N'-bis-(β-hydroxy-éthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

3. Composition selon la revendication 1 ou 2, **caractérisée par le fait que** le 2-chloro 6-méthyl 3-aminophénol et/ou le ou ses sels d'addition avec un acide représente de 0,0001 à 5% en poids du poids total de la composition tinctoriale.

4. Composition selon la revendication 3, **caractérisée par le fait que** le 2-chloro 6-méthyl 3-aminophénol et/ou le ou ses sels d'addition avec un acide représente de 0,005 à 3% en poids du poids total de la composition tinctoriale.

5. Composition selon l'une quelconque des revendications précédentes, **caractérisée par le fait que** la ou les bases d'oxydation représentent de 0,0005 à 12% en poids du poids total de la composition tinctoriale.

6. Composition selon la revendication 5, **caractérisée par le fait que** la ou les bases d'oxydation représentent de 0,005 à 6% en poids du poids total de la composition tinctoriale.

7. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates et que les sels d'addition avec une base sont ceux obtenus avec la soude, la potasse, l'ammoniaque ou les amines.

8. Composition selon l'une quelconque des revendications précédentes, **caractérisée par le fait qu'**elle présente un pH compris entre 3 et12.

9. Composition selon l'une quelconque des revendications précédentes, **caractérisée par le fait qu'**elle se présente sous forme de liquides, de crèmes ou de gels.

10. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 9, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

11. Procédé selon la revendication 10, **caractérisé par le fait que** l'agent oxydant présent dans la composition oxydante est choisi parmi le peroxyde d'hydrogène, le peroxyde d'urée, les bromates de métaux alcalins, les persels tels que les perborates et persulfates, les peracides.

12. Dispositif à plusieurs compartiments, ou "kit" de teinture à plusieurs compartiments, dont un premier compartiment renferme une composition tinctoriale telle que définie à l'une quelconque des revendications 1 à 9 et un second compartiment renferme une composition oxydante.

## Claims

1. Composition for the oxidation dyeing of human keratinous fibres, such as hair, **characterized in that** it comprises, in a medium appropriate for dyeing:
- 2-chloro-6-methyl-3-aminophenol and/or at least one of its addition salts with an acid, as coupler;
- and at least one oxidation base chosen from the double bases of following formula (II) and their addition salts with an acid: 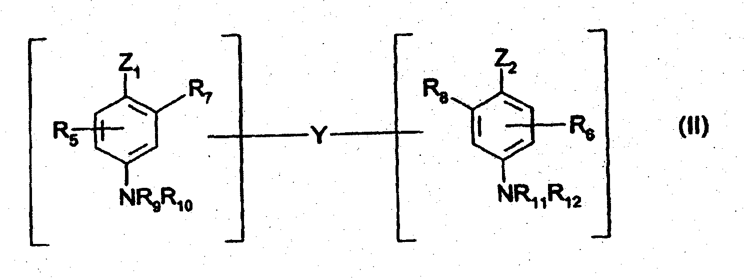
in which:
- Z₁ and Z₂, which are identical or different, represent a -NH₂ radical which can be substituted by a C₁-C₄ alkyl radical or by a connecting arm Y;
- the connecting arm Y represents a linear or branched alkylene chain comprising from 1 to 14 carbon atoms which can be interrupted by one or more nitrogenous groups and/or by one or more heteroatoms, such as oxygen, sulphur or nitrogen atoms, and which is optionally substituted by one or more hydroxyl or C₁-C₆ alkoxy radicals;
- R₅ and R₆ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a connecting arm Y;
- R₇, R₈, R₉, R₁₀, R₁₁ and R₁₂, which are identical or different, represent a hydrogen atom, a connecting arm Y or a C₁-C₄ alkyl radical;
it being understood that the compounds of formula (II) only comprise a single connecting arm Y per molecule;
the said composition being devoid of an additional coupler which would be chosen from 4-hydroxyindoline, 6-hydroxyindoline and their addition salts with an acid.

2. Composition according to Claim 1, **characterized in that** the double bases of formula (II) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)-tetramethylenediamine, N,N'-bis(4-methylaminophenyl)tetramethylenediamine, N,N'-diethyl-N,N'-bis(4'-amino-3'-methylphenyl)ethylenediamine and their addition salts with an acid.

3. Composition according to Claim 1 or 2, **characterized in that** 2-chloro-6-methyl-3-aminophenol and/or the addition salt(s) with an acid represent from 0.0001 to 5% by weight of the total weight of the dyeing composition.

4. Composition according to Claim 3, **characterized in that** 2-chloro-6-methyl-3-aminophenol and/or the addition salt(s) with an acid represent from 0.005 to 3% by weight of the total weight of the dyeing composition.

5. Composition according to any one of the preceding claims, **characterized in that** the oxidation base or bases represent from 0.0005 to 12% by weight of the total weight of the dyeing composition.

6. Composition according to Claim 5, **characterized in that** the oxidation base or bases represent from 0.005 to 6% by weight of the total weight of the dyeing composition.

7. Composition according to any one of the preceding claims, **characterized in that** the addition salts with an acid are chosen from hydrochlorides, hydrobromides, sulphates and tartrates, lactates and acetates and **in that** the addition salts with a base are those obtained with sodium hydroxide, potassium hydroxide, aqueous ammonia or amines.

8. Composition according to any one of the preceding claims, **characterized in that** it exhibits a pH of between 3 and 12.

9. Composition according to any one of the preceding claims, **characterized in that** it is provided in the form of liquids, creams or gels.

10. Process for dyeing keratinous fibres and in particular human keratinous fibres, such as hair, **characterized in that** at least one dyeing composition as defined in any one of Claims 1 to 9 is applied to these fibres and **in that** the colour is developed at acidic, neutral or alkaline pH using an oxidizing agent which is added only at the time of use to the dyeing composition or which is present in an oxidizing composition applied simultaneously or sequentially in a separate fashion.

11. Process according to Claim 10, **characterized in that** the oxidizing agent present in the oxidizing composition is chosen from hydrogen peroxide, urea hydrogen peroxide, alkali metal bromates, persalts, such as perborates and persulphates, or peracids.

12. Dyeing multi-compartment device or kit with several compartments, a first compartment of which includes a dyeing composition as defined in any one of Claims 1 to 9 and a second compartment of which includes an oxidizing composition.

## Patentansprüche

1. Zusammensetzung zum oxidativen Färben von menschlichen Keratinfasern, wie Haaren, **dadurch gekennzeichnet, dass** sie in einem zum Färben geeigneten Träger enthält:
- das 2-Chlor-6-methyl-3-aminophenol und/oder mindestens eines seiner Additionssalze mit einer Säure als Kuppler;
- und mindestens eine Oxidationsbase, die unter den Doppelbasen der folgenden Formel (II) und deren Additionssalzen mit einer Säure ausgewählt ist: 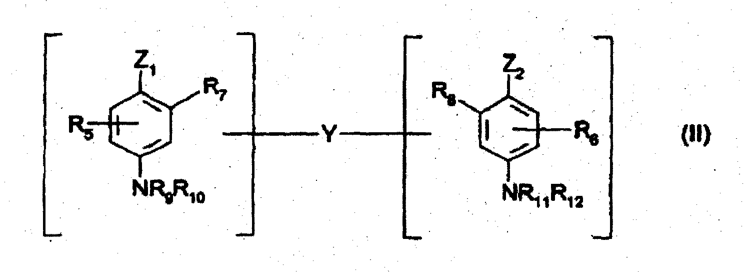
worin bedeuten:
- Z₁ und Z₂, die identisch oder voneinander verschieden sind, eine NH₂-Gruppe, die mit C₁₋₄-Alkyl oder einer Verbindungsgruppe Y substituiert sein kann,
- die Verbindungsgruppe Y eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen sein kann und gegebenenfalls mit einer oder mehreren Hydroxygruppen oder C₁₋₆-Alkoxygruppen substituiert sein kann,
- R₅ und R₆ ein Wasserstoffatom, ein Halogenatom, C₁₋₄-Alkyl, C₁₋₄-Monohydroxyalkyl, C₂₋₄-Polyhydroxyalkyl, C₁₋₄-Aminoalkyl oder eine Verbindungsgruppe Y,
- R₇, R₈, R₉, R₁₀, R₁₁ und R₁₂, die identisch oder voneinander verschieden sind, Wasserstoff, eine Verbindungsgruppe Y oder C₁₋₄-Alkyl,
mit der Maßgabe, dass die Verbindungen der Formel (II) nur eine Verbindungsgruppe Y pro Molekül aufweisen;
wobei die Zusammensetzung keinen weiteren Kuppler enthält, der unter 4-Hydroxyindolin, 6-Hydroxyindolin und deren Additionssalzen mit einer Säure ausgewählt ist.

2. Zusammensetzung nach Anspruch 1, **dadurch gekennzeichnet, dass** die Doppelbasen der Formel (II) unter N,N'-Bis(β-hydroxyethyl)-N,N'-bis(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis(4'-amino, 3'-methylphenyl)-ethylendiamin und deren Additionssalzen mit einer Säure ausgewählt sind.

3. Zusammensetzung nach Anspruch 1 oder 2, **dadurch gekennzeichnet, dass** das 2-Chlor-6-methyl-3-aminophenol und/ oder sein(e) Additionssalz(e) mit einer Säure 0,0001 bis 5 Gew.-% des Gesamtgewichts der Farbmittelzusammensetzung ausmachen.

4. Zusammensetzung nach Anspruch 3, **dadurch gekennzeichnet, dass** das 2-Chlor-6-methyl-3-aminophenol und/oder sein(e) Additionssalz(e) mit einer Säure 0,005 bis 3 Gew.-% des Gesamtgewichts der Farbmittelzusammensetzung ausmachen.

5. Zusammensetzung nach einem der vorhergehenden Ansprüche, **dadurch gekennzeichnet, dass** die Oxidationsbase(n) 0,0005 bis 12 Gew.-% des Gesamtgewichts der Farbmittelzusammensetzung ausmachen.

6. Zusammensetzung nach Anspruch 5, **dadurch gekennzeichnet, dass** die Oxidationsbase(n) 0,005 bis 6 Gew.-% des Gesamtgewichts der Farbmittelzusammensetzung ausmachen.

7. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten und die Additionssalze mit einer Base unter den Salzen ausgewählt sind, die mit Natriumhydroxid, Kaliumhydroxid, Ammoniak oder Aminen erhalten werden.

8. Zusammensetzung nach einem der vorhergehenden Ansprüche, **dadurch gekennzeichnet, dass** sie einen pH-Wert im Bereich von 3 bis 12 aufweist.

9. Zusammensetzung nach einem der vorhergehenden Ansprüche, **dadurch gekennzeichnet, dass** sie als Flüssigkeit, Creme oder Gel vorliegt.

10. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 9 aufgebracht wird und die Farbe bei einem sauren, neutralen oder alkalischen pH-Wert mit einem Oxidationsmittel gebildet wird, das unmittelbar bei der Anwendung zu der Farbmittelzusammensetzung gegeben wird oder das in einer oxidierenden Zusammensetzung vorliegt, die gleichzeitig oder getrennt davon anschließend aufgebracht wird.

11. Verfahren nach Anspruch 10, **dadurch gekennzeichnet, dass** das in der oxidierenden Zusammensetzung vorliegende Oxidationsmittel unter Wasserstoffperoxid, Harnstoffperoxid, Alkalimetallbromaten, Salzen von Persäuren, wie Perboraten und Persulfaten, und Persäuren ausgewählt ist.

12. Vorrichtung mit mehreren Abteilungen oder "Kit" zum Färben mit mehreren Abteilungen, **dadurch gekennzeichnet, dass** eine erste Abteilung die in den Ansprüchen 1 bis 9 definierte Farbmittelzusammensetzung und eine zweite Abteilung eine oxidierende Zusammensetzung enthält.
